# FASCICULE DE BREVET EUROPEEN

(11) **EP 1 844 329 B1**
(45) Date de publication et mention de la délivrance du brevet: **06.05.2009**
(21) Numéro de dépôt: 06709470.6
(22) Date de dépôt: 01.02.2006
(51) Int. Cl.: G01N 33/00

(54) **UTILISATION DE PINCES MOLECULAIRES COMME MATERIAUX SENSIBLES DANS DES CAPTEURS CHIMIQUES POUR LA DETECTION OU LE DOSAGE DE COMPOSES ORGANIQUES A L'ETAT DE VAPEURS**
VERWENDUNG MOLEKULARER CLIPS ALS EMPFINDLICHE MATERIALIEN IN CHEMISCHEN SENSOREN ZUM NACHWEIS ODER TESTEN ORGANISCHER VERBINDUNGEN IM DAMPFZUSTAND
USE OF MOLECULAR CLIPS AS SENSITIVE MATERIALS IN CHEMICAL SENSORS FOR DETECTING OR ASSAYING ORGANIC COMPOUNDS IN VAPOUR STATE

(30) Priorité: 02.02.2005 FR 0550304
(43) Date de publication de la demande: 17.10.2007
(73) Titulaire: COMMISSARIAT A L'ENERGIE ATOMIQUE, 75015 Paris (FR)
(72) Inventeur: HAIRAULT, Lionel, F-37150 Blere (FR); MONTMEAT, Pierre, F-37520 La Riche (FR); PASQUINET, Eric, F-37550 St Avertin (FR); BARBE, Jean-michel, F-21490 Bretigny (FR); BRANDES, Stéphane, F-21000 Dijon (FR); DENAT, Franck, F-21000 Dijon (FR); GROS, Claude, F-21800 Neuilly Les Dijon (FR); GUILARD, Roger, F-21121 Fontaine Les Dijon (FR)
(74) Mandataire: Poulin, Gérard
(86) Numéro de dépôt international: PCT/FR2006/050088
(87) Numéro de publication internationale: WO 2006/082343

(56) Documents cités:
- WO-A-03/011865
- US-A1- 2003 027 936
- US-A1- 2004 152 826
- BRANDÈS S ET AL: "NEW SYNTHESIS OF CYLINDRICAL MACROTICYCLIC LIGAND IN TETRAAZAMACROCYCLE SERIES" COMPTES RENDUS DE L ACADEMIE DES SCIENCES: SERIE II: MECANIQUE- PHYSIQUE-CHIMIE-ASTRONOMIE, EDITIONS SCIENTIFIQUES & MEDICALES ELSEVIER, FR, vol. 322, no. 11, 3 juin 1996 (1996-06-03), pages 827-833, XP000596908 ISSN: 1251-8069
- BELETSKAYA I P ET AL: "Synthesis of 1,8-bis(cyclam) and 1,8-bis(azacrown) substituted anthracenes by palladium-catalyzed arylation of cyclam" TETRAHEDRON LETTERS, ELSEVIER, AMSTERDAM, NL, vol. 43, no. 7, 11 février 2002 (2002-02-11), pages 1193-1196, XP004333883 ISSN: 0040-4039
- KADISH K M ET AL: "Synthesis, physicochemical and electrochemical properties of metal-metal bonded ruthenium corrole homodimers" JOURNAL OF ORGANOMETALLIC CHEMISTRY, ELSEVIER-SEQUOIA S.A. LAUSANNE, CH, vol. 652, no. 1-2, 1 juin 2002 (2002-06-01), pages 69-76, XP004361446 ISSN: 0022-328X
- BOLZE F ET AL: "Fine tuning of the photophysical properties of cofacial diporphyrins via the use of different spacers" JOURNAL OF ORGANOMETALLIC CHEMISTRY, ELSEVIER-SEQUOIA S.A. LAUSANNE, CH, vol. 643-644, 1 février 2002 (2002-02-01), pages 89-97, XP004339560 ISSN: 0022-328X
- ALI UMAR A ET AL: "Self-assembled monolayer of copper(II) meso-tetra(4-sulfanatophenyl) porphyrin as an optical gas sensor" SENSORS AND ACTUATORS B, ELSEVIER SEQUOIA S.A., LAUSANNE, CH, vol. 101, no. 1-2, 15 juin 2004 (2004-06-15), pages 231-235, XP004510316 ISSN: 0925-4005
- SPADAVECCHIA J ET AL: "Spin-coated thin films of metal porphyrin-phthalocyanine blend for an optochemical sensor of alcohol vapours" SENSORS AND ACTUATORS B, ELSEVIER SEQUOIA S.A., LAUSANNE, CH, vol. 100, no. 1-2, 1 juin 2004 (2004-06-01), pages 88-93, XP004509054 ISSN: 0925-4005
- DI NATALE C ET AL: "Characterization and design of porphyrins-based broad selectivity chemical sensors for electronic nose applications" SENSORS AND ACTUATORS B, ELSEVIER SEQUOIA S.A., LAUSANNE, CH, vol. 52, no. 1-2, 15 septembre 1998 (1998-09-15), pages 162-168, XP004157829 ISSN: 0925-4005
- TREDGOLD R H ET AL: "GAS SENSORS MADE FROM LANGMUIR-BLODGETT FILMS OF PORPHYRINS" IEE PROCEEDINGS I. SOLID- STATE & ELECTRON DEVICES, INSTITUTION OF ELECTRICAL ENGINEERS. STEVENAGE, GB, vol. 132 PART 1, no. 3, 1 juin 1985 (1985-06-01), pages 151-156, XP000570796 ISSN: 0956-3776
- ANDERSSON M ET AL: "Development of a ChemFET sensor with molecular films of porphyrins as sensitive layer" SENSORS AND ACTUATORS B, ELSEVIER SEQUOIA S.A., LAUSANNE, CH, vol. 77, no. 1-2, 15 juin 2001 (2001-06-15), pages 567-571, XP004246610 ISSN: 0925-4005
- EWA PACHOLSKA ET AL.: "New route to a face.to face biscorrole free-base and the corresponding heterobimetallic copper (III)-silver(III) complex" DALTON TRANSACTIONS, 16 septembre 2004 (2004-09-16), pages 3181-3183, XP002351448 The royal Society of Chemistry
- SÉBASTIEN FAURE ET AL.: "Role of the Spacer in the singlet-Singlet energy Transfer Mechanism in cofacial Bisporphyrins" J. AM. CHEM. SOC., vol. 126, no. 4, 1 juin 2004 (2004-06-01), pages 1253-1261, XP002351449 columbus Ohio
- MOHAMMED LACHKAR ET AL.: "synthesis of new Macrotricyclic ligands where two cyclam Rings are in a face-to-face conformation. characterization of their Dicopper(II) and Dinickel(II) complexes" INORGANIC CHEMISTRY, vol. 37, no. 7, 13 mars 1998 (1998-03-13), pages 1575-1584, XP002351450 Columbus Ohio
- STÉPHANE BRANDÉS ET AL.: "Synthesis of Macropolycyclic Ligands based on Tetraazacycloalkanes." EUR. J. ORG. CHEM, 1998, pages 2349-2360, XP002351451 Wiley-VCH Verlag Weinheim
- JEAN-MICHEL BARBE ET AL.: "Metallocorroles as sensing components for gas sensors: remarquable affinity and selectivity of cobalt(III)corroles for CO vs O2 and N2." DALTON TRANSACTIONS, 23 mars 2004 (2004-03-23), pages 1208-1214, XP002351452 The Royal sociey of Chemistry
- SHIGERU KUROSAWA ET AL.: "Gas Sorption to Plasma-Polymerized copper Phthalocyanine film formed on a piezoelectric Crystal." ANALYTICAL CHEMISTRY, vol. 62, no. 4, 15 février 1990 (1990-02-15), pages 353-359, XP002351453 columbus ohio
- M.G. BARON ET AL.: "Luminescent porphyrin thin films for NOX sensing" SENSORS AND ACTUATORS B, no. 11, 1993, pages 195-199, XP002351457 Elsevier Sequoia

## Description

### DOMAINE TECHNIQUE

La présente invention se rapporte à l'utilisation de pinces moléculaires en tant que matériaux sensibles dans des capteurs chimiques destinés à détecter ou à doser des composés organiques se trouvant à l'état de vapeurs, et plus spécifiquement des composés nitrés comme les nitroaromatiques (nitrobenzène, dinitrobenzène, trinitrobenzène, nitrotoluène, dinitrotoluène, trinitrotoluène, ...), les nitramines, les nitrosamines et les esters nitriques.

De tels capteurs sont notamment utiles pour la détection d'explosifs, que ce soit en vue d'assurer la sécurité de lieux publics comme les aéroports, de contrôler la licéité de marchandises en circulation sur un territoire, de lutter contre le terrorisme, de procéder à des opérations de désarmement, de localiser des mines antipersonnel ou encore de dépolluer des sites industriels ou militaires.

Ils sont également utiles pour la protection de l'environnement, en particulier pour le contrôle et la surveillance de la pollution atmosphérique et de la qualité d'ambiances plus ou moins confinées, ainsi que pour la surveillance à des fins sécuritaires, de sites industriels fabriquant, stockant et/ou manipulant des composés nitrés.

### ÉTAT DE LA TECHNIQUE ANTÉRIEURE

La détection d'explosifs est un problème d'intérêt crucial, notamment en matière de sécurité civile.

A l'heure actuelle, plusieurs méthodes sont utilisées pour détecter des vapeurs de composés nitrés entrant dans la constitution des explosifs, comme l'emploi de chiens "*renifleurs*" dressés et entraînés à cet effet, l'analyse en laboratoire, par exemple par chromatographie couplée à un spectromètre de masse ou à un détecteur à capture d'électrons, d'échantillons prélevés sur site, ou encore la détection infrarouge.

Ces méthodes font, d'une manière générale, preuve d'une grande sensibilité, ce qui est primordial en matière de détection d'explosifs compte tenu de la très faible concentration en vapeurs de composés nitrés qui règne au voisinage d'un explosif. Elles ne donnent toutefois pas totalement satisfaction.

Ainsi, l'utilisation de chiens "*renifleurs*" présente l'inconvénient de nécessiter une longue formation des chiens et de leurs maîtres et d'être inadaptée à des opérations prolongées en raison de ce que la durée d'attention des chiens est limitée.

Quant aux autres méthodes, l'encombrement des appareillages qu'elles utilisent, leur consommation d'énergie et leurs coûts de mise en oeuvre s'opposent au développement de systèmes de détection aisément transportables et autonomes et, partant, aptes à être utilisés sur tout type de sites.

Depuis quelques années, le développement de capteurs capables de détecter en temps réel des espèces chimiques gazeuses est en plein essor. Le fonctionnement de ces capteurs est basé sur l'utilisation d'un film d'un matériau sensible, c'est-à-dire d'un matériau dont au moins une propriété physique P (masse, température, conductivité électrique, absorbance, fluorescence, ...) est modifiée au contact des molécules gazeuses recherchées, qui revêt un système apte à mesurer en temps réel toute variation de cette propriété physique et de mettre ainsi en évidence la présence des molécules gazeuses recherchées.

Les avantages des capteurs chimiques par rapport aux méthodes précitées sont multiples : instantanéité des résultats, possibilité de miniaturisation et, donc, portabilité, maniabilité et autonomie importante, faibles coûts de fabrication et d'exploitation, etc.

Toutefois, il est évident que leurs performances sont extrêmement variables selon la nature du matériau sensible utilisé.

A ce jour, un certain nombre de travaux visant à rechercher des matériaux sensibles pour la détection de composés nitrés gazeux, et plus particulièrement de composés nitroaromatiques, a été réalisé. A ainsi été étudiée la possibilité d'utiliser des polysiloxanes, des polyéthylèneglycols, des adsorbants du type charbon, des composés organiques cycliques (phtalocyanine de cuivre, cyclodextrines, cavitands), des dendrimères et des composés fluorescents.

Par ailleurs, le piégeage de composés par des "*pinces moléculaires*", c'est-à-dire des molécules composées de deux branches, généralement aromatiques, reliées l'une à l'autre par un groupe espaceur, a été proposé à la fin des années 1970.

La possibilité de piéger des composés aromatiques ou nitroaromatiques en milieu liquide *via* différents types de pinces a été depuis démontrée par un certain nombre d'auteurs, mais ces derniers ne se sont pas intéressés au piégeage de ces composés en milieu gazeux.

Or, dans le cadre de leurs travaux sur le développement de capteurs chimiques destinés plus spécialement à la détection d'explosifs, les Inventeurs ont constaté que des pinces moléculaires de type bismacrocycles face-à-face réagissent avec une très grande sensibilité à la présence de vapeurs de composés nitrés, et plus généralement de vapeurs de composés organiques, et sont donc susceptibles de constituer des matériaux sensibles de choix pour la détection ou le dosage de ces composés lorsqu'ils se trouvent à l'état de vapeurs.

Et c'est cette constatation qui est à la base de l'invention.

### EXPOSÉ DE L'INVENTION

L'invention a, donc, pour objet l'utilisation d'au moins un composé répondant à la formule générale (I) ci-après : 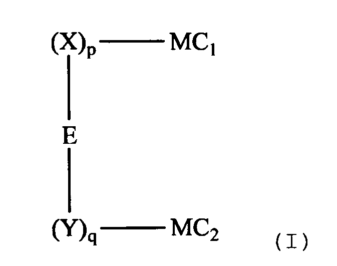 dans laquelle :
MC₁ et MC₂, qui peuvent être identiques ou différents, représentent des macrocycles ;
p et q, qui peuvent être identiques ou différents, valent 0 ou 1 ;
X et Y, qui peuvent être identiques ou différents, représentent des groupes alkylènes comportant de 1 à 10 atomes de carbone, éventuellement substitués ; tandis que
E représente un groupe espaceur cyclique ou hétérocyclique, éventuellement substitué ;
   et dans laquelle MC₁ et MC₂ sont disposés face-à-face ;
   en tant que matériau sensible dans un capteur chimique pour détecter ou doser un composé organique à l'état de vapeurs.

Dans le cadre de la présente invention, on entend généralement par "*macrocycle*", une molécule organique qui peut être constituée d'un seul cycle ou de plusieurs cycles reliés les uns aux autres, soit directement par une liaison simple, soit par l'intermédiaire d'un atome ou d'un groupe formant pont, et dont le cycle ou l'ensemble des cycles comprend, de préférence, de 8 à 60 atomes de carbone et un ou plusieurs hétéroatomes, cette molécule pouvant être de plus métallée, c'est-à-dire être liée à un atome de métal, et/ou substituée.

Par ailleurs, l'expression "*disposés face-à-face*" signifie que les macrocycles MC₁ et MC₂ sont orientés dans le composé de sorte à être l'un en face de l'autre mais cela ne signifie pas que ces macrocycles sont nécessairement parallèles l'un à l'autre.

Ainsi, les macrocycles MC₁ et MC₂ peuvent notamment être choisis, indépendamment l'un de l'autre, parmi les porphyrines, les phtalocyanines, les naphtalocyanines, les sapphyrines, les corroles, les corrolazines et les polyamines macrocycliques du type polyazamacrocycles ou dioxopolyazamacrocycles, métallées et non métallées, substituées et non substituées.

Le métal auquel peuvent être liés les macrocycles MC₁ et/ou MC₂, lorsque ceux-ci sont métallés, peut être *a priori* n'importe quel élément considéré comme un métal au sens de la classification périodique des éléments, encore connue sous le nom de classification de Mendeleiev, à savoir un métal alcalin comme le lithium, un métal alcalino-terreux comme le magnésium, un métal de transition comme le fer, le cobalt, le zinc, le cuivre, le nickel, le manganèse, le chrome ou le titane, ou encore un mét-al des colonnes III, IV et V de cette classification comme le plomb.

Ce métal peut, de plus, être lié à un atome d'halogène ou à un groupe hydroxyle, c'est-à-dire qu'il peut être sous la forme d'un halogénure ou d'un hydroxyde métallique.

Le ou les substituants portés par les macrocycles MC₁ et/ou MC₂, lorsque ceux-ci sont substitués, peuvent être choisis parmi :
* les groupes hydrocarbonés, linéaires, ramifiés ou cycliques, saturés ou insaturés, comprenant de 1 à 100 atomes de carbone et comportant éventuellement un ou plusieurs hétéroatomes et/ou une ou plusieurs fonctions chimiques comprenant au moins un hétéroatome et/ou un ou plusieurs groupes aromatiques ou hétéroaromatiques ;
* les fonctions chimiques comprenant au moins un hétéroatome ; et
* les groupes aromatiques ou hétéroaromatiques, éventuellement substitués.

Lorsque ce ou ces substituants consistent en un groupe hydrocarboné et que ce groupe comprend au moins deux atomes de carbone et comporte un ou plusieurs hétéroatomes et/ou une ou plusieurs fonctions chimiques comprenant au moins un hétéroatome et/ou un ou plusieurs groupes aromatiques ou hétéroaromatiques, alors ce ou ces hétéroatomes, cette ou ces fonctions chimiques et ce ou ces groupes aromatiques ou hétéroaromatiques peuvent aussi bien former pont à l'intérieur de ce groupe hydrocarboné qu'être portés latéralement par lui ou encore se situer à son extrémité.

Dans ce qui précède et ce qui suit, on entend par "*hétéroatome*", tout atome autre que de carbone ou d'hydrogène comme, par exemple, un atome d'oxygène, de soufre, d'azote, de fluor, de chlore, de phosphore ou encore de bore, les atomes d'oxygène, d'azote, de soufre et d'halogène étant préférés.

On entend par "*fonction chimique comprenant au moins un hétéroatome*", toute fonction chimique comportant un ou plusieurs atomes autres que de carbone ou d'hydrogène et, notamment, une fonction comportant un ou plusieurs atomes d'oxygène, de soufre, d'azote et/ou d'halogène. Cette fonction chimique peut, en particulier, être choisie parmi les fonctions -COOH, -COOR, -CHO, -CO, -OH, -OR, -SH, -SR, -SO₂R, -NH₂, -NHR, -NRR', -CONH₂, -CONHR, -CONRR', -C(Hal)₃, -OC(Hal)₃, -C(O)Hal, -CN, -COR, -COOCOR et phénol, dans lesquelles :
R représente un groupe hydrocarboné, linéaire, ramifié ou cyclique, saturé ou insaturé, en C₁ à C₁₀₀, ou bien une liaison simple dans le cas où ladite fonction chimique forme pont dans un groupe hydrocarboné ;
R' représente un groupe hydrocarboné, linéaire, ramifié ou cyclique, saturé ou insaturé, en C₁ à C₁₀₀, ce groupe pouvant être identique ou différent du groupe hydrocarboné représenté par R' ; tandis que
Hal représente un atome d'halogène, par exemple un atome de fluor, de chlore ou de brome.

On entend par "*groupe aromatique*", tout groupe hydrocarboné constitué d'un ou plusieurs cycles insaturés en C₃ à C₆ et comportant des doubles liaisons conjuguées, et par "*groupe hétéroaromatique*", tout groupe aromatique tel qu'il vient d'être défini, mais comprenant, dans le cycle ou au moins l'un des cycles qui le constituent, un ou plusieurs hétéroatomes. A titre d'exemples de groupes aromatiques susceptibles d'être utilisés, on peut citer les groupes cyclo-pentadiényle, phényle, benzyle, biphényle, phényl-acétylényle, pyrényle ou anthracényle, tandis qu'à titre d'exemples de groupes hétéroaromatiques, on peut citer les groupes furanyle, pyrrolyle, thiophényle, oxazolyle, pyrazolyle, thiazolyle, imidazolyle, triazolyle, pyridinyle, pyranyle, quinoléinyle, pyrazinyle et pyrimidinyle.

Lorsqu'un tel groupe aromatique ou hétéroaromatique est substitué, alors il comporte, de préférence, une ou plusieurs fonctions chimiques comprenant au moins un hétéroatome telles que celles mentionnées ci-dessus.

Le groupe espaceur E peut être, d'une manière générale, tout groupe cyclique ou hétérocyclique, les Inventeurs ayant, en effet, constaté que, quelle que soit la nature du groupe espaceur, les composés de formule générale (I) sont aptes à être utilisés comme matériaux sensibles dans des capteurs gravimétriques, c'est-à-dire des capteurs dont le fonctionnement est basé sur une variation de la masse de ces matériaux.

Ainsi, ce groupe espaceur peut être un groupe mono- ou polycyclique, saturé ou insaturé, voire aromatique ou hétéroaromatique. A titre d'exemples de groupes espaceurs, on peut citer les groupes phényle, pyrényle, anthracényle, naphtalényle, dibenzofuranyle, biphénylényle, dibenzothiophényle, xanthényle, métallocényle (par exemple, ferrocényle), *ortho*-, méta- ou para-xylényle, carbazolyle, acridinyle, phénazinyle, phénoxazinyle, binaphtyle, phénothiazinyle, fluorényle, diphényl-étheroxyde ou encore calix[n]arényle où n est un nombre entier allant de 4 à 12.

Toutefois, il est également possible de jouer sur le choix du groupe espaceur pour conférer aux composés de formule générale (I) des propriétés physiques particulières, susceptibles d'être modifiées en présence des composés que l'on souhaite détecter et aisément mesurables, rendant les composés de formule générale (I) aptes à servir également de matériaux sensibles dans des capteurs autres que gravimétriques.

Ainsi, par exemple, un groupe espaceur comportant des propriétés de fluorescence tel qu'un groupe anthracényle, xanthényle ou encore acridinyle, permet de réaliser un capteur à fluorescence, tandis qu'un groupe espaceur du type dibenzothiophényle permet de réaliser un capteur résistif, c'est-à-dire un capteur dont le fonctionnement est basé sur une variation de la conductivité électrique du matériau sensible.

Comme précédemment indiqué, lorsqu'ils sont présents, les groupes alkylènes X et Y peuvent être substitués.

Il en est de même pour le groupe espaceur E.

Conformément à l'invention, le ou les substituants susceptibles d'être portés par X, Y et/ou E peuvent être choisis parmi :
* les groupes hydrocarbonés linéaires, ramifiés ou cycliques, saturés ou insaturés, en C₁ à C₃₀, comportant éventuellement un ou plusieurs hétéroatomes et/ou une ou plusieurs fonctions chimiques comprenant un ou plusieurs hétéroatomes et/ou un ou plusieurs groupes aromatiques ou hétéroaromatiques substitués ou non ;
* les fonctions chimiques comportant au moins un hétéroatome ; et
* les groupes aromatiques ou hétéroaromatiques substitués ou non.

Là également, lorsque ce ou ces substituants consistent en un groupe hydrocarboné et que ce groupe comprend au moins deux atomes de carbone et comporte un ou plusieurs hétéroatomes et/ou une ou plusieurs fonctions chimiques et/ou un ou plusieurs groupes aromatiques ou hétéroaromatiques, alors ce ou ces hétéroatomes, cette ou ces fonctions chimiques et ce ou ces groupes aromatiques ou hétéroaromatiques peuvent aussi bien former pont à l'intérieur de ce groupe hydrocarboné qu'être portés latéralement par lui ou encore se situer à son extrémité.

Parmi les composés utiles selon l'invention, on préfère notamment ceux répondant à la formule générale (I) dans laquelle :
MC₁ et MC₂, qui peuvent être identiques ou différents, représentent deux porphyrines ou deux phtalocyanines ou deux naphtalocyanines ou deux sapphyrines ou deux corroles ou deux corrolazines ou deux polyamines macrocycliques, métallées ou non métallées, substituées ou non substituées ;
p et q sont identiques, X et Y sont identiques ; tandis que E a la même signification que précédemment.

Plus spécifiquement, on préfère ceux qui répondent à la formule générale (I) dans laquelle :
MC₁ et MC₂ représentent :
   - soit deux porphyrines choisies parmi celles répondant aux formules (II) et (III) ci-après : 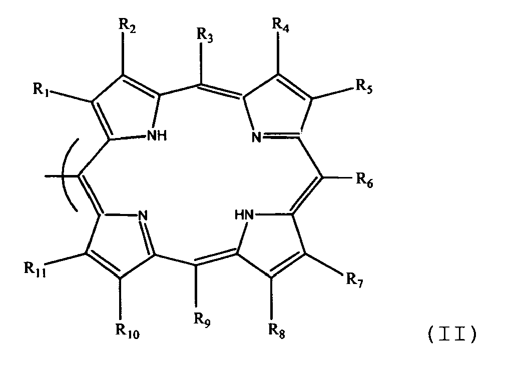 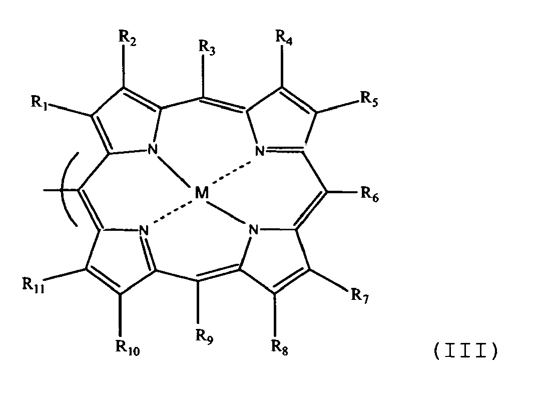
   - soit deux dioxopolyazamacrocycles choisis parmi ceux répondant aux formules (IV) et (V) ci-après : 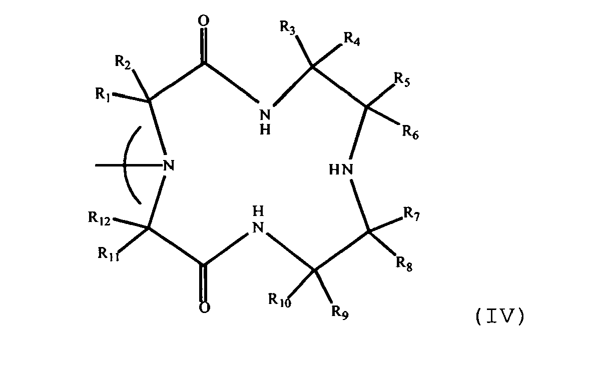 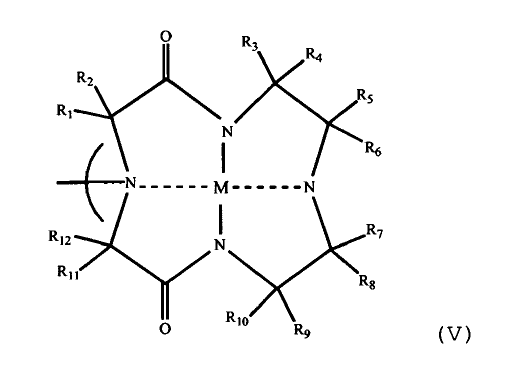
   - soit deux polyazamacrocycles choisis parmi ceux répondant aux formules (VI) et (VII) ci-après : 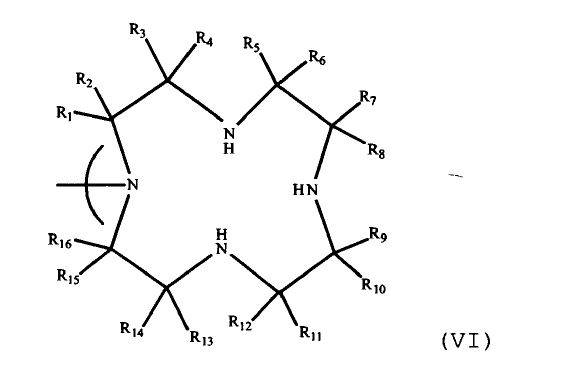 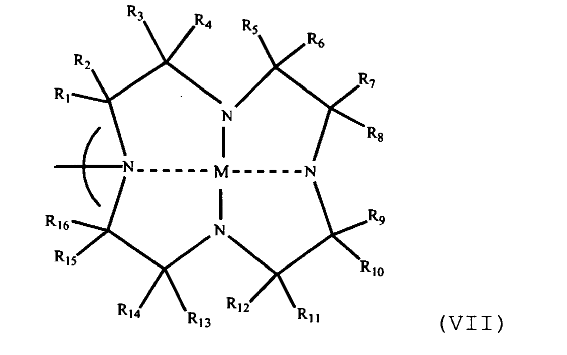 où R¹, R², R³, R⁴, R⁵, R⁶, R⁷, R⁸, R⁹, R¹⁰, R¹¹, R¹², R¹³, R¹⁴, R¹⁵, R¹⁶ et R¹⁷, identiques ou différents, représentent un atome d'hydrogène ou un groupe hydrocarboné, linéaire ou ramifié, saturé ou insaturé, en C₁ à C₁₀, et M représente un métal choisi parmi le lithium, le magnésium, le fer, le cobalt, le zinc, le cuivre, le nickel, le manganèse, le chrome, le titane et le plomb ;
p et q sont identiques, X et Y sont identiques ; tandis que E a la même signification que précédemment.

De tels composés sont, par exemple :
* les bisporphyrines de formules particulières (IIIa), (IIIb) et (IIIc) ci-après : 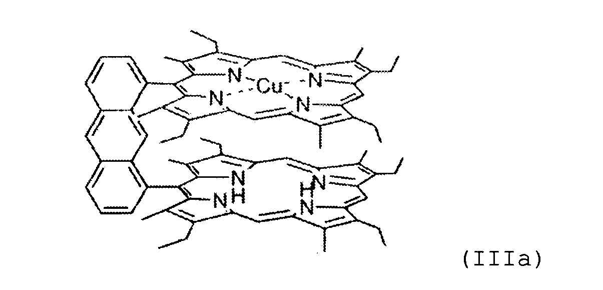 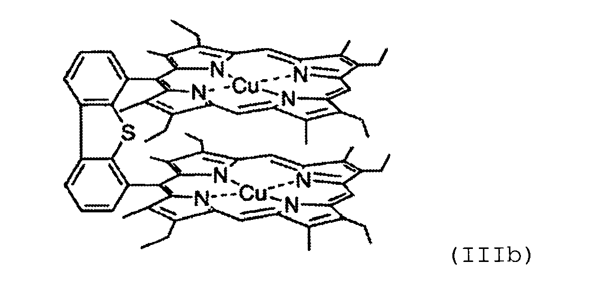 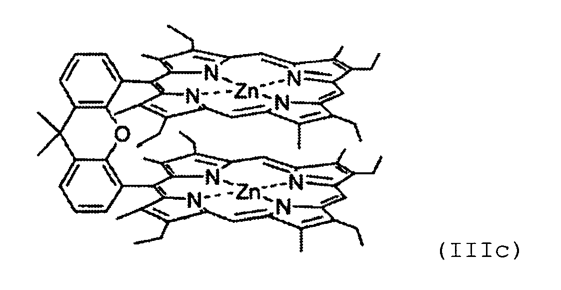
* le bispolyazamacrocycle de formule particulière (IVa) ci-après : 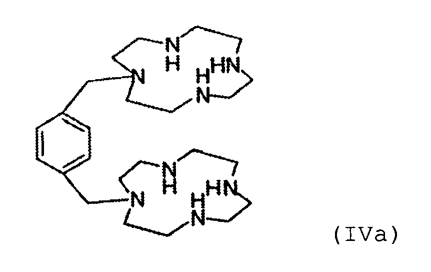
* le bisdioxopolyazamacrocycle de formule particulière (VIa) ci-après :

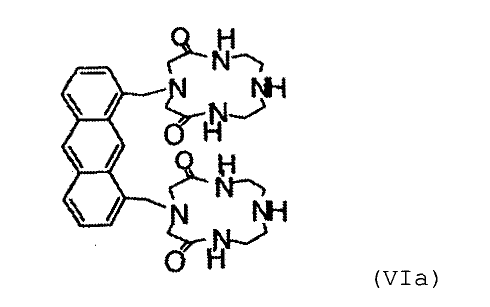

Les composés de formule générale (I) peuvent être synthétisés par des procédés décrits dans la littérature.

En particulier, les bisporphyrines non métallées peuvent être obtenues par condensation de 4 motifs pyrroliques α libres sur un groupe de formule (VIII) ci-après : 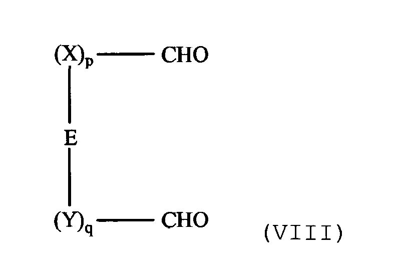 dans laquelle X, Y, p, q, E ont la même signification que précédemment et les deux groupes -CHO sont disposés face-à-face, cette condensation étant réalisée dans l'éthanol à reflux en présence d'une quantité catalytique d'acide chlorhydrique, ce qui permet d'obtenir des bis(dipyrrylméthanes) porteurs de 4 fonctions esters éthyliques en positions α pyrroliques. Ces esters sont ensuite hydrolysés par une solution alcaline, par exemple de diéthylèneglycol à 100°C, puis la température du milieu réactionnel est progressivement amenée à 190°C pour induire une décarboxylation douce des tétraacides précédemment obtenus mais non isolés. Puis, on soumet les bispyrrylméthanes tétra α libres à une cyclisation avec un dipyrrylméthane (à raison de deux molécules de dipyrrylméthane par molécule de bispyrrylméthane), en présence d'acide *para*-toluène sulfonique, suivie d'une oxydation par l'*ortho*-chloranil. L'isolement des bisporphyrines résultantes et leur purification par chromatographie sur colonne est facilitée grâce à une métallation par le zinc que l'on fait suivre ensuite d'une démétallation par un traitement en milieu acide.

Les bisporphyrines bihomométallées du type de celles répondant aux formules particulières (IIIb) et (IIIc) peuvent être obtenues en soumettant les bisporphyrines correspondantes non métallées à une bismétallation, au reflux par exemple d'un mélange dichlorométhane/méthanol et en présence d'un excès du sel métallique destiné à être complexé, par exemple sous forme d'acétate métallique.

Les bisporphyrines monométallées du type de celle répondant à la formule particulière (IIIa) peuvent être obtenues en soumettant les bisporphyrines correspondantes non métallées à deux monométallations successives, la première avec un métal destiné à servir de groupement protecteur pour la porphyrine que l'on ne souhaite pas métaller, par exemple du zinc sous forme d'acétate de zinc, la seconde avec le métal destiné à être complexé par l'autre porphyrine, puis en éliminant le premier métal par un traitement en milieu acide.

Les bisporphyrines bihétérométallées peuvent être obtenues d'une manière analogue aux bisporphyrines, à ceci près que le premier métal n'est pas éliminé.

Les bispolyazamacrocycles du type de celui répondant à la formule particulière (IVa) peuvent être obtenus par condensation des tétraazacycloalcanes correspondants triprotégés par des groupes protecteurs, par exemple *tert*-butyloxycarbonyle (t-Boc), sur un groupe :
* soit de formule (IX) ci-après : 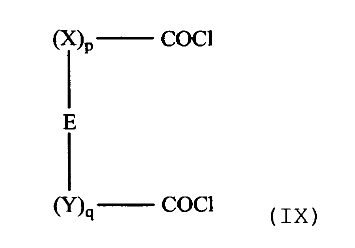
dans laquelle X, Y, p, q, E ont la même signification que précédemment et les deux groupes -COCl sont disposés face-à-face, auquel cas cette condensation est réalisée dans le tétrahydrofuranne en présence de triéthylamine, pendant 1 heure à température ambiante, puis, après hydrolyse du milieu réactionnel et extraction au chloroforme, le brut réactionnel est chromatographié sur gel de silice pour conduire aux bisamides intermédiaires ;
* soit de formule (X) ci-après : 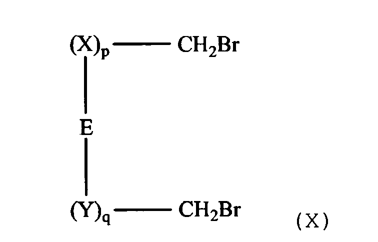
dans laquelle X, Y, p, q, E ont la même signification que précédemment et les deux groupes -CH₂Br sont disposés face-à-face, auquel cas la condensation est réalisée au reflux de l'acétonitrile en présence de carbonate de potassium, pendant 48 heures, puis, après évaporation du solvant et chromatographie sur silice, les bisamides intermédiaires sont obtenus.

Dans tous les cas, les bisamides intermédiaires sont réduits par un excès de borane dans le tétrahydrofuranne et les groupements protecteurs t-Boc sont éliminés par hydrolyse acide avec de l'acide chlorhydrique.

Les bisdioxopolyazamacrocycles du type de celui répondant à la formule particulière (VIa) peuvent, eux, être obtenus par un procédé analogue à celui qui vient d'être décrit mais à partir des dioxopolyazamacrocycles correspondants dont une fonction amine est protégée par un groupe t-Boc.

Conformément à l'invention, le composé de formule générale (I) est présent dans le capteur de préférence sous la forme d'un film mince qui recouvre l'une ou les deux faces d'un substrat convenablement choisi en fonction de la propriété physique dont les variations sont destinées à être mesurées par ce capteur.

En variante, le composé de formule générale (I) peut également être présent dans le capteur sous la forme d'un objet massif comme, par exemple, un cylindre présentant une certaine porosité de sorte à rendre accessible aux composés que l'on souhaite détecter l'ensemble des molécules du composé de formule générale (I).

Lorsque ce dernier se présente sous la forme d'un film mince, ce film présente, de préférence, une épaisseur de 10 angströms à 100 microns.

Un tel film peut notamment être obtenu par dépôt par pulvérisation, par dépôt à la tournette ("*spin coating*" en langue anglo-saxonne), par dépôt à la goutte ou encore par dépôt par sublimation, toutes ces techniques de dépôt étant bien connues de l'homme du métier.

Le substrat ainsi que le système de mesure du capteur sont choisis en fonction de la propriété physique du composé de formule générale (I) dont les variations induites par la présence des composés à détecter sont destinées à être mesurées par le capteur.

En l'espèce, les variations de masse des composés de formule générale (I) et les variations de fluorescence de ces composés, lorsqu'ils présentent des propriétés de fluorescence, se sont révélées particulièrement intéressantes à mesurer.

Aussi, le capteur est-il, de préférence, un capteur gravimétrique ou un capteur à fluorescence.

A titre d'exemples de capteurs gravimétriques, on peut citer les capteurs du type à microbalance à quartz, les capteurs à ondes de surface, plus connus sous la terminologie anglo-saxonne "SAW" pour "Surface Acoustic Wave", tels que les capteurs à ondes de Love et les capteurs à ondes de Lamb, ainsi que les microleviers.

Parmi les capteurs gravimétriques, on préfère plus particulièrement les capteurs à microbalance à quartz. Ce type de capteurs, dont le principe de fonctionnement a été décrit par J.A.O. Sanchez-Pedrono et al. dans Anal. Chem. Acta, vol. 182, 1986, 285, comprend, schématiquement, un substrat piézo-électrique (ou résonateur), généralement un cristal de quartz recouvert sur ses deux faces d'une couche métallique, par exemple d'or ou de platine, servant d'électrode. Le matériau sensible recouvrant l'une ou les deux faces du substrat, toute variation de masse de ce matériau se traduit par une variation de la fréquence de vibration du substrat.

Bien entendu, il est également possible d'utiliser un composé de formule générale (I) comme matériau sensible dans des capteurs conçus pour mesurer des variations d'une propriété physique autre que la masse et la fluorescence comme, par exemple, des capteurs résistifs basés sur la mesure de variations de conductivité électrique ou des capteurs optiques basés sur la mesure de variations d'absorbance dans le domaine UV-visible ou encore de longueur d'onde dans le domaine des infrarouges.

Par ailleurs, il est également possible de réunir au sein d'un même dispositif ou "*multicapteur*", plusieurs capteurs élémentaires comprenant des matériaux sensibles différents les uns des autres, ou munis de substrats et de systèmes de mesure différents les uns des autres comme, par exemple, un ou plusieurs capteurs gravimétriques et/ou un ou plusieurs capteurs à fluorescence, l'essentiel étant que l'un au moins de ces capteurs comprenne un composé de formule générale (I).

Des capteurs comportant un composé de formule générale (I) en tant que matériau sensible se sont révélés aptes à détecter ou doser avec une très grande sensibilité de nombreux composés organiques à l'état de vapeurs.

Ces composés organiques sont notamment les composés nitrés, dont ils sont capables de détecter la présence à des concentrations de l'ordre du ppm (partie par million), voire du ppb (partie par billion), voire encore, dans certains cas, du ppt (partie par trillion), et en particulier les composés nitroaromatiques, les nitramines, les nitrosamines et les esters nitriques.

A titre d'exemples de composés nitroaromatiques, on peut citer le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'amino-dinitrotoluène, le dinitrotrifluorométhylbenzène, le chlorodinitrotrifluorométhylbenzène, l'hexanitro-stilbène ou encore le trinitrophénol (ou acide picrique).

Les nitramines sont, elles, par exemple la cyclotétraméthylènetétranitramine (ou octogène), la cyclotriméthylènetrinitramine (ou hexogène) et la trinitrophénylméthylnitramine (ou tétryle), tandis que les nitrosamines sont, par exemple, la nitrosodiméthylamine.

Quant aux esters nitriques, il s'agit, par exemple, de pentrite, de dinitrate d'éthylèneglycol, de dinitrate de diéthylèneglycol, de nitroglycérine ou de nitroguanidine.

Toutefois, les composés organiques susceptibles d'être détectés ou dosés par les capteurs sont également les composés organiques volatiles, et en particulier les cétones, les alcools, les solvants chlorés et les composés aromatiques du type toluène.

Outre les avantages sus-mentionnés, des capteurs comportant un composé de formule générale (I) en tant que matériau sensible se sont révélés présenter encore d'autres avantages comme :
* une rapidité de réponse et une reproductibilité de cette réponse ;
* une stabilité des performances dans le temps et, partant, une durée de vie très satisfaisante ;
* une aptitude à fonctionner en continu ;
* un coût de fabrication compatible avec une production de capteurs en série, une très faible quantité de composé de formule générale (I) (c'est-à-dire, en pratique de quelques mg) étant nécessaire pour la fabrication d'un capteur, et
* la possibilité d'être miniaturisés et, partant, d'être aisément transportables et manipulables sur tout type de sites.

Ils sont donc particulièrement utiles pour détecter des explosifs, notamment dans des lieux publics.

L'invention sera mieux comprise à la lumière du complément de description, qui se rapporte à des exemples d'utilisation de différents exemples de composés utiles selon l'invention, sous la forme de films minces, en tant que matériaux sensibles dans des capteurs à microbalance à quartz et à fluorescence.

Bien entendu, ces exemples ne sont donnés qu'à titre d'illustration de l'objet de l'invention et ne constituent en aucun cas une limitation de cet objet.

### BRÈVE DESCRIPTION DES DESSINS

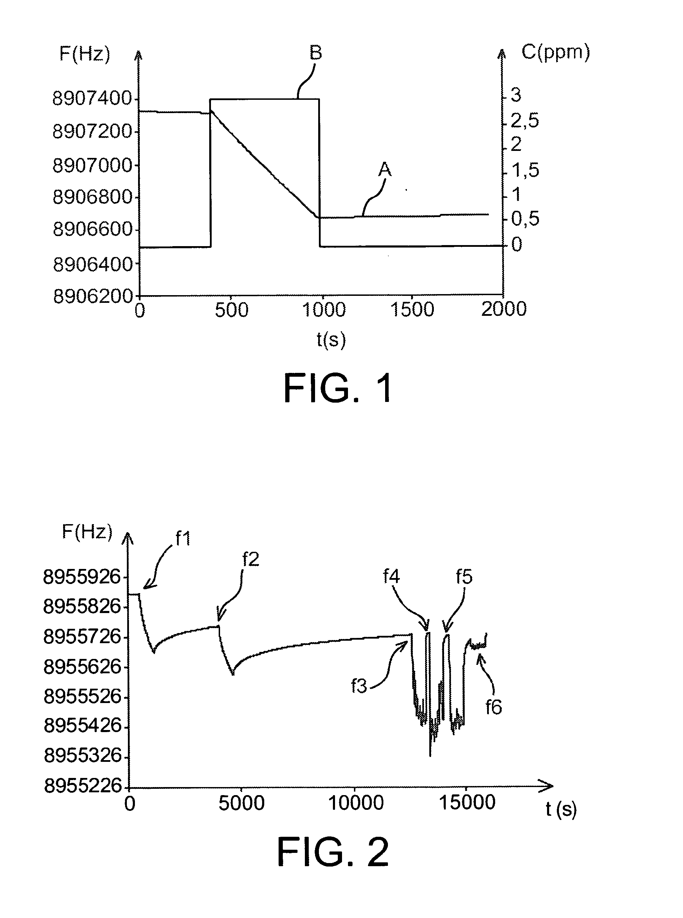

La figure 1 représente l'évolution de la fréquence de vibration du quartz d'un capteur à microbalance à quartz comprenant un film mince d'un premier exemple de composé utile selon l'invention, lorsque ce capteur est exposé successivement à de l'air et à des vapeurs de 2,4-dinitrotrifluorométhoxybenzène (DNTFMB).

La figure 2 représente l'évolution de la fréquence de vibration du quartz d'un capteur à microbalance à quartz comprenant un film mince d'un deuxième exemple de composé utile selon l'invention, lorsque ce capteur est exposé successivement à de l'air et à des vapeurs de DNTFMB, de dichlorométhane, de méthyléthylcétone, de toluène et d'éthanol.

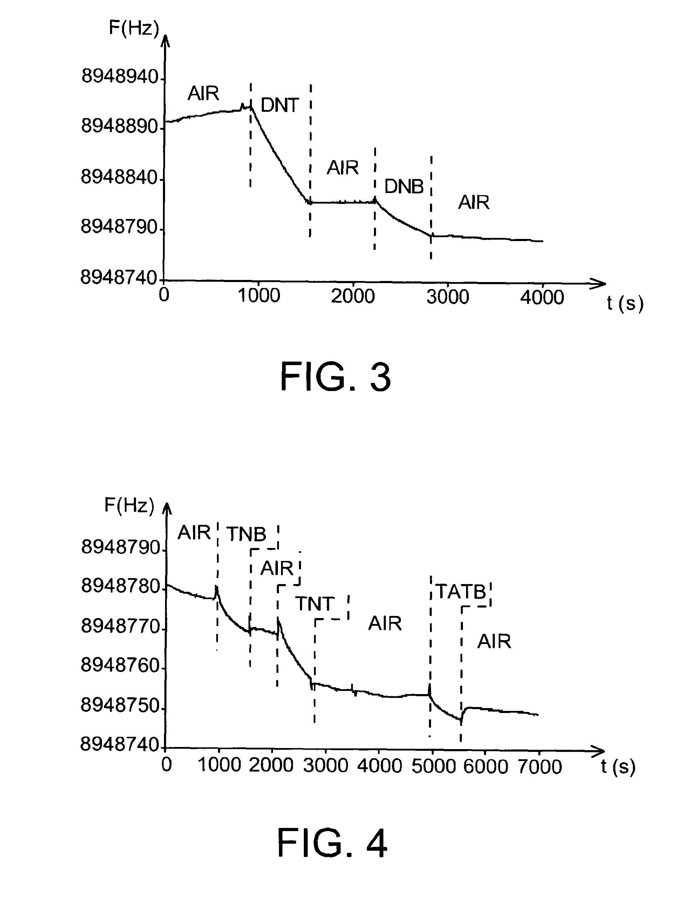

La figure 3 représente l'évolution de la fréquence de vibration du quartz d'un capteur à microbalance à quartz comprenant un film mince du premier exemple de composé utile selon l'invention, lorsque ce capteur est exposé successivement à de l'air et à des vapeurs de 2,4-dinitrotoluène (DNT) et de dinitrobenzène (DNB).

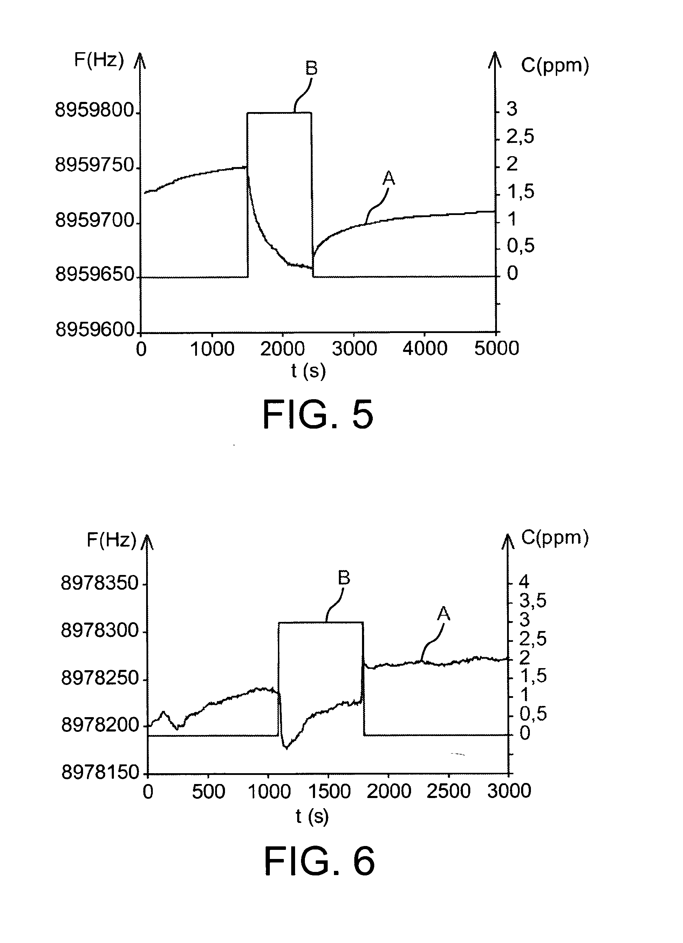

La figure 4 représente l'évolution de la fréquence de vibration du quartz d'un capteur à microbalance à quartz comprenant un film mince du premier exemple de composé utile selon l'invention, lorsque ce capteur est exposé successivement à de l'air et à des vapeurs de trinitrobenzène (TNB), de trinitrotoluène (TNT) et de triaminotrinitrobenzène (TATB). La figure 5 représente l'évolution de la fréquence de vibration du quartz d'un capteur à microbalance à quartz comprenant un film mince d'un troisième exemple de composé utile selon l'invention, lorsque ce capteur est exposé successivement à de l'air et à des vapeurs de DNTFMB.

La figure 6 représente l'évolution de la fréquence de vibration du quartz d'un capteur à microbalance à quartz comprenant un film mince d'un quatrième exemple de composé utile selon l'invention, lorsque ce capteur est exposé successivement à de l'air et à des vapeurs de DNTFMB.

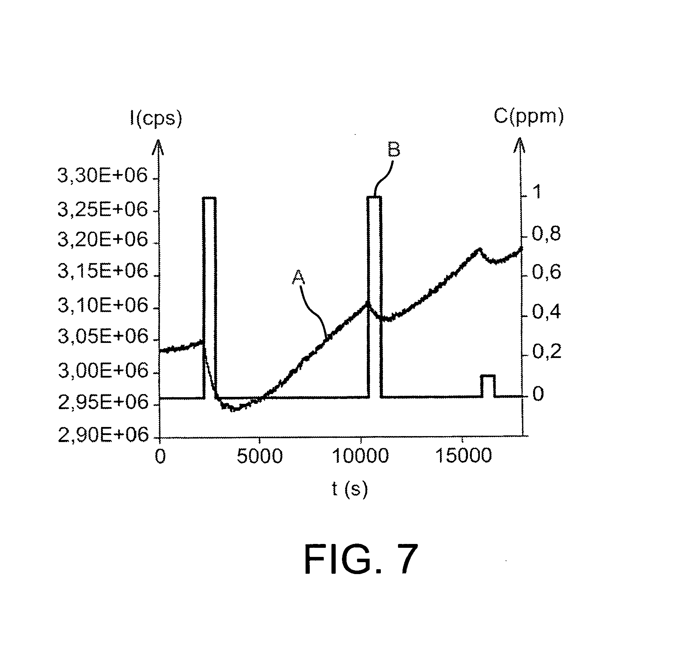

La figure 7 représente l'évolution de l'intensité de fluorescence émise par un capteur à fluorescence comprenant un film mince d'un cinquième exemple de composé utile selon l'invention, lorsque ce capteur est exposé successivement à de l'air et à des vapeurs de DNTFMB.

### EXPOSÉ DÉTAILLÉ DE MODES DE REALISATION PARTICULIERS

### Exemple 1 : Détection d'un composé nitré (DNTFMB) par un capteur à microbalance à quartz

Dans cet exemple, on utilise un capteur à microbalance à quartz comprenant un quartz de coupe AT, de fréquence de vibration de 9 MHz, recouvert de deux électrodes de mesure circulaires en or (modèle QA9RA-50, AMETEK PRECISION INSTRUMENTS), et portant sur ses deux faces un film mince de la bisporphyrine de formule particulière (IIIa) représentée ci-avant.

Le dépôt de ce film est réalisé par sublimation de la bisporphyrine, à une température de 100°C et sous un vide partiel de 4.10⁻⁵ mbar, jusqu'à obtenir une variation de la fréquence de vibration du quartz de 10 kHz.

Le capteur est exposé successivement à :
- de l'air pendant 7 minutes,
- du DNTFMB à une concentration de 3 ppm dans de l'air pendant 10 minutes, et
- de l'air pendant 20 minutes,
l'air et le DNTFMB étant à température ambiante (25°C).

La figure 1 illustre l'évolution de la fréquence de vibration du quartz au cours de ces expositions. Sur cette figure, la courbe A représente les valeurs de la fréquence de vibration (F) du quartz, exprimées en hertz (Hz), en fonction du temps (t), exprimé en secondes (s), tandis que la courbe B représente les valeurs de la concentration en DNTFMB (C), exprimées en ppm, également en fonction du temps.

### Exemple 2 : Détection de différents types de composés organiques par un capteur à microbalance à quartz

Dans cet exemple, on utilise un capteur à microbalance à quartz comprenant un quartz identique à celui du capteur utilisé dans l'exemple 1, mais dont le quartz est recouvert sur ses deux faces d'un film mince de la bisporphyrine de formule particulière (IIIb) représentée ci-avant.

Le dépôt de ce film est réalisé par sublimation de la bisporphyrine, à une température de 100°C et sous un vide partiel de 2.10⁻⁵ mbar, jusqu'à obtenir une variation de la fréquence de vibration du quartz de 10 kHz.

Le capteur est exposé successivement à :
- de l'air pendant 8 minutes,
- du DNTFMB à une concentration de 3 ppm dans de l'air pendant 10 minutes,
- de l'air pendant 50 minutes,
- du DNTFMB à une concentration de 3 ppm dans de l'air pendant 10 minutes,
- de l'air pendant 130 minutes,
- du dichlorométhane à une concentration de 580 000 ppm dans de l'air pendant 10 minutes,
- de l'air pendant 3 minutes,
- de la méthyléthylcétone à une concentration de 126 000 ppm dans de l'air pendant 10 minutes,
- de l'air pendant 6 minutes,
- du toluène à une concentration de 38 000 ppm dans de l'air pendant 10 minutes,
- de l'air pendant 8 minutes,
- de l'éthanol à une concentration de 79 000 ppm dans de l'air pendant 10 minutes, et
- de l'air pendant 2 minutes,
l'air, le DNTFMB, le dichlorométhane, la méthyléthylcétone, le toluène et l'éthanol étant à température ambiante (25°C).

La figure 2 illustre l'évolution de la fréquence de vibration du quartz au cours de ces expositions, sous la forme d'une courbe représentant les valeurs de la fréquence de vibration (F) du quartz, exprimées en hertz (Hz), en fonction du temps (t), exprimé en secondes (s), les flèches f1 et f2 signalant les deux expositions au DNTFMB, la flèche f3 l'exposition au dichlorométhane, la flèche f4 l'exposition à la méthyléthylcétone, la flèche f5 l'exposition au toluène et la flèche f6 celle à l'éthanol.

Les diminutions de fréquence de vibration enregistrées lors des expositions aux vapeurs des composés organiques sont les suivantes :
DNTFMB 1^{ère} exposition : -179 Hz
DNTFMB 2^{ème} exposition : -139 Hz
Dichlorométhane : -291 Hz
Méthyléthylcétone : -293 Hz
Toluène : -275 Hz
Ethanol : -20 Hz.

### Exemple 3 : Détection de deux composés nitrés (DNT et DNB) par un troisième capteur à microbalance à quartz

Dans cet exemple, on utilise un capteur à microbalance à quartz comprenant un quartz identique à celui du capteur utilisé dans l'exemple 1 et dont le quartz est recouvert sur ses deux faces d'un film mince de la bisporphyrine de formule particulière (IIIa) représentée ci-avant.

Le dépôt de ce film est également réalisé par sublimation de ladite bisporphyrine à une température de 100°C, mais sous vide partiel de 2.10⁻⁵ bar et jusqu'à obtenir une variation de la fréquence de vibration du quartz de 20 kHz.

Le capteur est exposé successivement à :
- de l'air pendant 15 minutes,
- du DNT à une concentration de 285 ppb dans de l'air pendant 10 minutes,
- de l'air pendant 11 minutes,
- du DNB à une concentration de 5 ppm dans de l'air pendant 10 minutes, et
- de l'air pendant 20 minutes,
l'air, le DNT et le DNB étant à température ambiante (25°C).

La figure 3 illustre l'évolution de la fréquence de vibration du quartz au cours de ces expositions, sous la forme d'une courbe représentant les valeurs de la fréquence de vibration (F) du quartz, exprimées en hertz (Hz), en fonction du temps (t), exprimé en secondes (s).

### Exemple 4 : Détection de trois composés nitrés (TNB, TNT et TATB) par un capteur à microbalance à quartz

Dans cet exemple, on utilise un capteur identique à celui utilisé dans l'exemple 3.

Ce capteur est exposé successivement à :
- de l'air pendant 15 minutes,
- du TNB à une concentration de 285 ppb dans de l'air pendant 10 minutes,
- de l'air pendant 8 minutes,
- du TNT à une concentration de 7 ppb dans de l'air pendant 12 minutes,
- de l'air pendant 36 minutes,
- du TATB à une concentration inférieure à 1 ppt dans de l'air pendant 10 minutes, et
- de l'air pendant 25 minutes, l'air, le TNB, le TNT et le TATB étant à température ambiante (25°C).

La figure 4 illustre l'évolution de la fréquence de vibration du quartz au cours de ces expositions, sous la forme d'une courbe représentant les valeurs de la fréquence de vibration (F) du quartz, exprimées en hertz (Hz), en fonction du temps (t), exprimé en secondes (s).

### Exemple 5 : Détection d'un composé nitré (DNTFMB) par un capteur à microbalance à quartz

Dans cet exemple, on utilise un capteur à microbalance à quartz comprenant un quartz identique à celui du capteur utilisé dans l'exemple 1, mais dont le quartz est recouvert sur ses deux faces d'un film mince de la bisporphyrine de formule particulière (IIIc) représentée ci-avant.

Le dépôt de ce film est réalisé par sublimation de la bisporphyrine, à une température de 100°C et sous vide partiel de 2.10⁻⁵ mbar, jusqu'à obtenir une variation de la fréquence de vibration du quartz de 10 kHz.

Le capteur est exposé successivement à :
- de l'air pendant 25 minutes,
- du DNTFMB à une concentration de 3 ppm dans de l'air pendant 10 minutes,
- de l'air pendant 30 minutes, l'air et le DNTFMB étant à température ambiante (25°C).

La figure 5 illustre l'évolution de la fréquence de vibration du quartz au cours de ces expositions. Sur cette figure, la courbe A représente les valeurs de la fréquence de vibration (F) du quartz, exprimées en hertz (Hz), en fonction du temps (t), exprimé en secondes (s), tandis que la courbe B représente les valeurs de la concentration en DNTFMB (C), exprimées en ppm, également en fonction du temps.

### Exemple 6 : Détection d'un composé nitré (DNTFMB) par un capteur à microbalance à quartz

Dans cet exemple, on utilise un capteur à microbalance à quartz comprenant un quartz identique à celui du capteur utilisé dans l'exemple 1, mais dont le quartz est recouvert sur ses deux faces d'un film mince du bispolyazamacrocycle de formule particulière (IVa) représentée ci-avant.

Le dépôt de ce film est réalisé en effectuant sur chaque face du quartz 60 pulvérisations de 0,4 seconde chacune d'une solution du bispolyazamacrocycle dans le méthanol, de concentration égale à 5 g/L, de sorte à obtenir une variation de la fréquence de vibration du quartz de 10 kHz.

Le capteur est exposé successivement à :
- de l'air pendant 18 minutes,
- du DNTFMB à une concentration de 3 ppm dans de l'air pendant 10 minutes, et
- de l'air pendant 20 minutes, l'air et le DNTFMB étant à température ambiante (25°C).

La figure 6 illustre l'évolution de la fréquence de vibration du quartz au cours de ces expositions. Sur cette figure, la courbe A représente les valeurs de la fréquence de vibration (F) du quartz, exprimées en hertz (Hz), en fonction du temps (t), exprimé en secondes (s), tandis que la courbe B représente les valeurs de la concentration en DNTFMB (C), exprimées en ppm, également en fonction du temps.

### Exemple 7 : Détection d'un composé nitré (DNTFMB) par un capteur à fluorescence

Dans cet exemple, on utilise un capteur dont le fonctionnement est basé sur la variation de l'intensité de la fluorescence émise par le matériau sensible que comporte ce capteur en présence d'un composé nitré.

En l'espèce, le matériau sensible est constitué par le dioxomacrocycle de formule particulière (VIa) représentée ci-avant, sous forme d'un film mince qui recouvre l'une des faces d'un substrat en verre de qualité optique (TUET et BIECHELIN), obtenu par 14 pulvérisations de 0,4 seconde chacune d'une solution de ce composé dans le méthanol, de concentration égale à 5 g/L.

Le film mince ainsi obtenu présente une intensité de fluorescence de 30.10⁵ coups/seconde (λ_{émission} : 511 nm ; λ_{excitation} : 392 nm) telle que mesurée au moyen d'un fluorimètre FluoroMax-3 de la société JOBIN YVON, en régime dynamique dans une cellule balayée à 24 L/h et thermostatée à 25°C.

Le capteur est exposé successivement à :
- de l'azote pur pendant 35 minutes,
- du DNTFMB à une concentration de 1 ppm dans de l'azote pendant 10 minutes,
- de l'air pendant 48 minutes,
- du DNTFMB à une concentration de 1 ppm dans de l'azote pendant 10 minutes,
- de l'air pendant 120 minutes,
- du DNTFMB à une concentration de 0,1 ppm dans de l'azote pendant 10 minutes, et
- de l'air pendant 18 minutes, l'azote, le DNTFMB et l'air étant à température ambiante (25°C).

La figure 7 illustre l'évolution de l'intensité de fluorescence émise par le capteur au cours de ces expositions, cette intensité de fluorescence étant mesurée dans les mêmes conditions que celles précisées ci-dessus. Sur cette figure, la courbe A représente les valeurs de l'intensité de fluorescence (I), exprimées en coups/seconde (cps), en fonction du temps (t), exprimé en secondes (s), tandis que la courbe B représente les valeurs de la concentration en DNTFMB (C), exprimées en ppm, également en fonction du temps.

Les exemples ci-avant montrent que des capteurs à microbalance à quartz ou à fluorescence comprenant un composé de formule générale (I) comme matériau sensible, sont capables de détecter avec une très grande sensibilité non seulement des vapeurs de composés nitrés comme des vapeurs de DNTFMB, DNB, TNB, TNT ou de TATB, mais également des vapeurs d'autres composés organiques volatils comme, par exemple, ceux classiquement utilisés comme solvants : composés chlorés, aromatiques, cétones et alcools. La détection de composés organiques appartenant à différentes classes est ainsi possible avec ces capteurs.

Ces exemples montrent aussi que la réponse de ces capteurs est à la fois réversible et reproductible.

## Revendications

1. Utilisation d'au moins un composé répondant à la formule générale (I) ci-après : 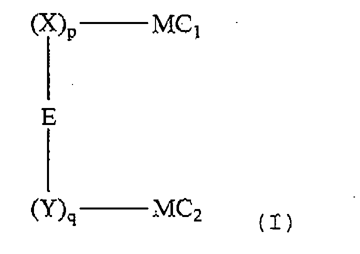 dans laquelle :
MC₁ et MC₂, qui peuvent être identiques ou différents, représentent des macrocycles, ces macrocycles étant constitués d'un seul cycle ou de plusieurs cycles reliés les uns aux autres par une liaison simple ou par l'intermédiaire d'un atome ou d'un groupe formant pont, et dont le cycle ou l'ensemble des cycles comprend de 8 à 60 atomes de carbone et un ou plusieurs hétéroatomes ;
p et q, qui peuvent être identiques ou différents, valent C ou 1 ;
X et Y, qui peuvent être identiques ou différents, sont des groupes alkylènes comportant de 1 à 10 atomes de carbone ; tandis que
E représente un groupe espaceur cyclique ou hétérocyclique ;
et dans laquelle MC₁ et MC₂ sont disposés face-à-face ; en tant que matériau sensible dans un capteur chimique pour détecter ou doser un ou plusieurs composés organiques à l'état de vapeurs.

2. Utilisation selon la revendication 1,
dans laquelle les macrocycles MC₁ et MC₂ sont choisis parmi les porphyrines, les phtalocyanines, les naphtalocyanines, les sapphyrines, les corroles, les corrolazines, les polyamines macrocycliques, métallées et non métallées, substituées et non substituées.

3. Utilisation selon la revendication 2,
dans laquelle les porphyrines, phtalocyanines, naphtalocyanines, sapphyrines, corroles, corrolazines et polyamines macrocycliques métallées renferment du lithium, du magnésium, fer, du cobalt, du zinc, du cuivre, du nickel, du manganèse, du chrome, du titane ou du plomb.

4. Utilisation selon l'une quelconque des revendications précédentes, dans laquelle, dans la formule générale (I), le groupe espaceur E est choisi parmi les groupes phényle, pyrényle, anthracényle, naphtalényle, dibenzofuranyle, biphénylényle, dibenzothiophényle, xanthényle, métallocényle, *ortho-, méta-* ou *para*-xylényle, carbazolyle, acridinyle, phénazinyle, phénoxazinyle, binaphtyle, phénothiazinyle, fluorényle, diphénylétheroxyde et calix[n]arényle où n est un nombre entier allant de 4 à 12.

5. Utilisation selon l'une quelconque des revendications précédentes, dans laquelle le composé répond à la formule générale (I) dans laquelle :
MC₁ et MC₂, qui peuvent être identiques ou différents, représentent deux porphyrines ou deux phtalocyanines ou deux naphtalocyanines ou deux sapphyrines ou deux corroles ou deux corrolazines ou deux polyamines macrocycliques, métallées ou non métallées, substituées ou non substituées ;
p et q sont identiques, X et Y sont identiques ; tandis que E a la même signification que précédemment.

6. Utilisation selon la revendication 5,
dans laquelle le composé répond à la formule générale (I) dans laquelle :
MC₁ et MC₂ représentent :
• soit deux porphyrines choisies parmi celles répondant aux formules (II) et (III) ci-après : 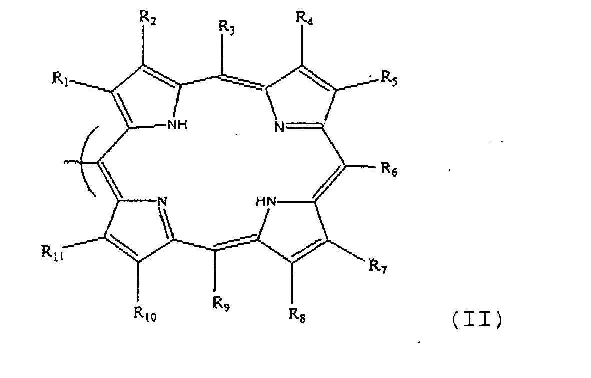 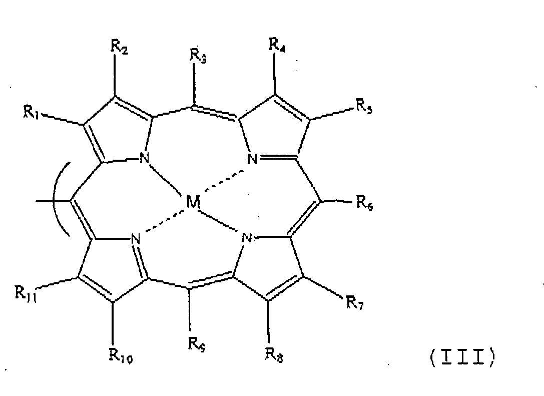
• soit deux dioxopolyazamacrocycles choisis parmi ceux répondant aux formules (IV) et (V) ci-après : 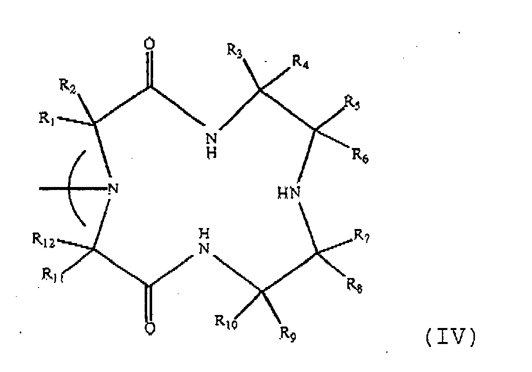 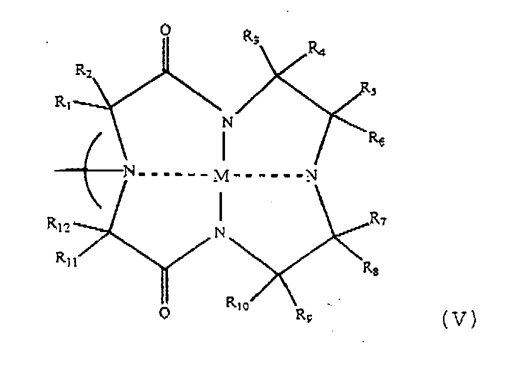
• soit deux polyazamacrocycles choisis parmi ceux répondant aux formules (VI) et (VII) ci-après : 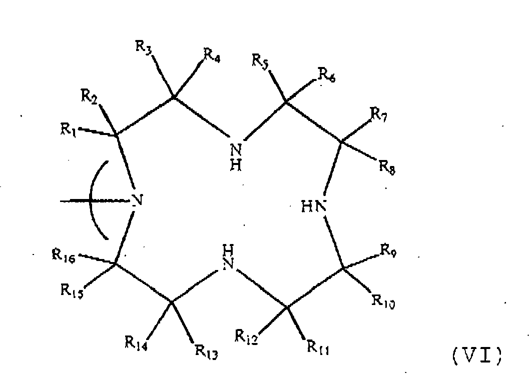 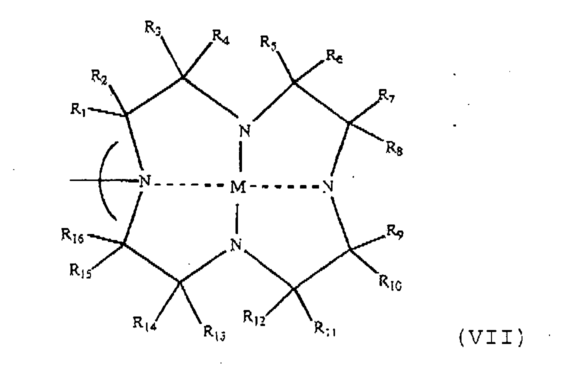 où R¹, R², R³, R⁴, R⁵, R⁶, R⁷, R⁸, R⁹ R¹⁰, R¹¹, R¹², R¹³, R¹⁴, R¹⁵, R¹⁶ et R¹⁷, identiques ou différents, représentent un atome d'hydrogène ou un groupe hydrocarboné, linéaire ou ramifié, saturé ou insaturé, en C₁ à C₁₀, et M représente un métal choisi parmi le lithium, le magnésium, le fer, le cobalt, le zinc, le cuivre, le nickel, le manganèse, le chrome, le titane et le plomb ;
p et q sont identiques, X et Y sont identiques ; tandis que E a la même signification que précédemment.

7. Utilisation selon la revendication 6,
dans laquelle le composé répond à l'une quelconque des formules particulières (IIIa), (IIIb), (IIIc), (IVa) et (VIa) ci-après :

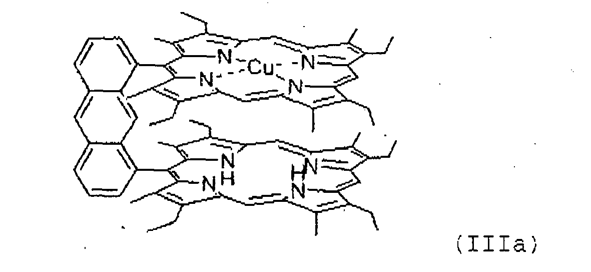

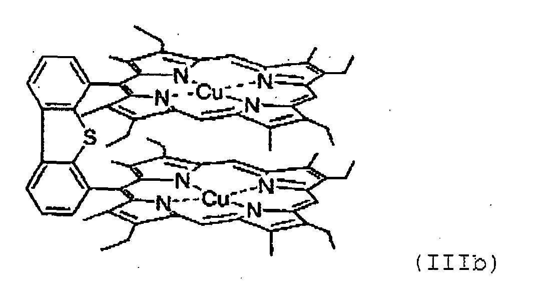

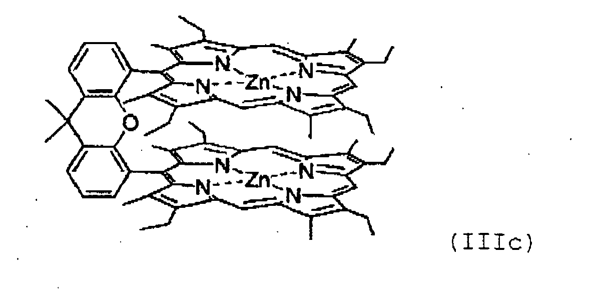

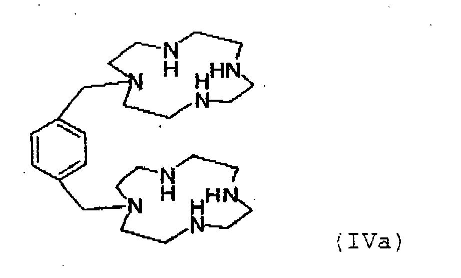

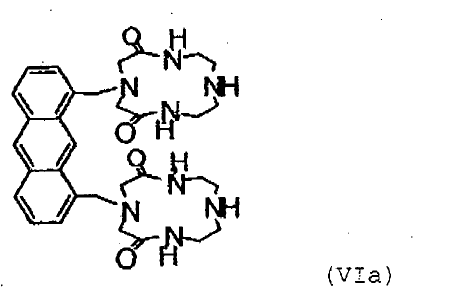

8. Utilisation selon l'une quelconque des revendications précédentes, dans laquelle le composé est présent dans le capteur sous la forme d'un film mince recouvrant l'une ou les deux faces d'un substrat.

9. Utilisation selon la revendication 8,
dans laquelle le film mince mesure de 10 angströms à 100 microns d'épaisseur.

10. Utilisation selon l'une quelconque des revendications précédentes, dans laquelle le capteur est un capteur gravimétrique.

11. Utilisation selon la revendication 10,
dans laquelle le capteur gravimétrique est un capteur à microbalance à quartz.

12. Utilisation selon l'une quelconque des revendications 1 à 9, dans laquelle le capteur est un capteur à fluorescence.

13. Utilisation selon l'une quelconque des revendications 1 à 9, dans laquelle le capteur est un multicapteur qui comprend un ou plusieurs capteurs gravimétriques et/ou un ou plusieurs capteurs à fluorescence, l'un au moins de ces capteurs comprenant un composé répondant à la formule générale (I).

14. Utilisation selon l'une quelconque des revendications précédentes, dans laquelle le ou les composés organiques à détecter ou doser sont un ou des composés nitrés.

15. Utilisation selon la revendication 14, dans laquelle le ou les composés nitrés sont choisis parmi les composés nitroaromatiques, les nitramines, les nitrosamines et les esters nitriques.

16. Utilisation selon la revendication 15, dans laquelle le ou les composés nitrés sont choisis parmi le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène, le trinitrophénol, la cyclotétraméthylène-tétranitramine, la cyclotriméthylènetrinitramine, la trinitrophénylméthylnitramine, la nitrosodiméthylamine, le pentrite, le dinitrate d'éthylèneglycol, le dinitrate de diéthylèneglycol, la nitroglycérine ou la nitroguanidine.

17. Utilisation selon l'une quelconque des revendications précédentes, dans laquelle le capteur est destiné à détecter des explosifs.

18. Utilisation selon l'une quelconque des revendications 1 à 13, dans laquelle le ou les compostés organiques à détecter ou doser sont un ou des composés organiques volatiles;

19. Utilisation selon la revendication 18,
dans laquelle le ou les composés organiques volatiles sont choisis parmi les cétones, les alcools, les solvants chlorés et les composés aromatiques.

## Claims

1. Use of at least one compound corresponding to the general formula (I) below: 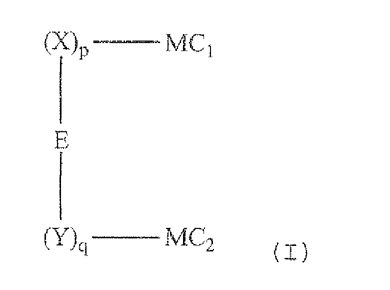 in which:
MC₁ and MC₂, which may be identical or different, represent macrocycles, these macrocycles being composed of a singe ring or of several rings joined together by a simple bond or via a bridging atom or group, and of which the ring or the set of rings comprises from 8 to 60 carbon atoms and one or more heteroatoms;
p and q, which may be identical or different, are equal to 0 or 1;
X and Y, which may be identical or different, are alkylene groups comprising from 1 to 10 carbon atoms; while
E represents a cyclic or heterocyclic spacer group;
and in which MC₁ and MC₂ are positioned facing each other;
as a sensitive material in a chemical sensor for detecting or assaying one or more organic compounds in the vapour state.

2. Use according to Claim 1, in which the macrocycles MC₁ and MC₂ are chosen from metalled and non-metalled, substituted and unsubstituted porphyrins, phthalocyanins, naphthalocyanins, sapphyrins, corroles, corrolazines and macrocyclic polyamines.

3. Use according to Claim 2, in which the metalled porphyrins, phthalocyanins, naphthalocyanins, sapphyrins, corroles, corrolazines and macrocyclic polyamines contain lithium, magnesium, iron, cobalt, zinc, copper, nickel, manganese, chromium, titanium or lead.

4. Use according to any one of the preceding claims, in which, in the general formula (I), the spacer group E is chosen from phenyl, pyrenyl, anthracenyl, naphthalenyl, dibenzofuranyl, biphenylenyl, dibenzothiophenyl, xanthenyl, metallocenyl, *ortho*-, *met*- or *para*-xylenyl, carbazolyl, acridinyl, phenazinyl, phenoxazinyl, binaphthyl, phenothiazinyl, fluorenyl, diphenyl ether oxide and calix[n]arenyl groups where n is an integer ranging from 4 to 12.

5. Use according to any one of the preceding claims, in which the compound corresponds to the general formula (I) in which:
MC₁ and MC₂, which may be identical or different, represent two porphyrins or two phthalocyanins or two naphthalocyanins or two sapphyrins or two corroles or two corrolazines or two macrocyclic polyamines which are metalled or non-metalled, substituted or unsubstituted;
p and q are identical, X and Y are identical; while E has the same meaning as before.

6. Use according to Claim 5, in which the compound corresponds to the general formula (I) in which:
MC₁ and MC₂ represent:
• either two porphyrins chosen from those corresponding to formulae (II) and (III) below: 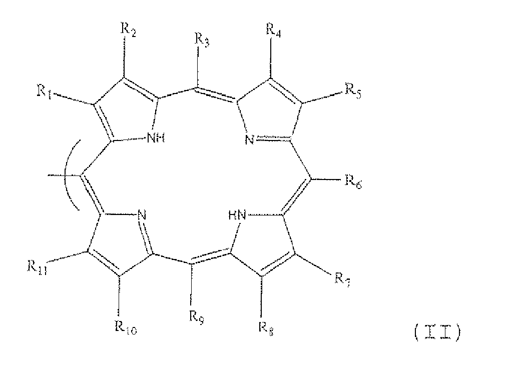 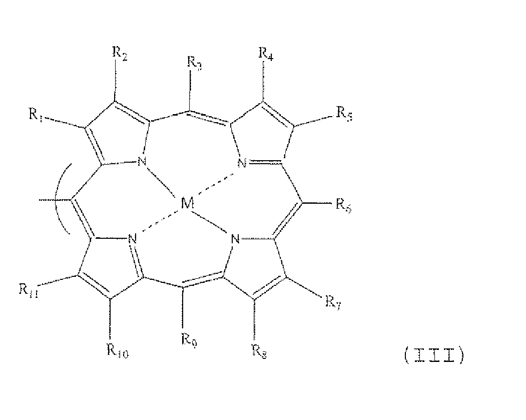
• or two dioxopolyazamacrocycles chosen from those corresponding to formulae (IV) and (V) below: 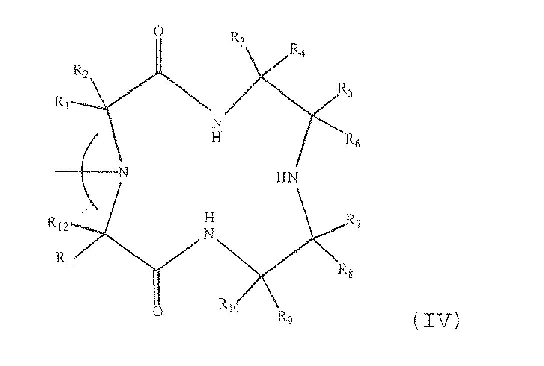 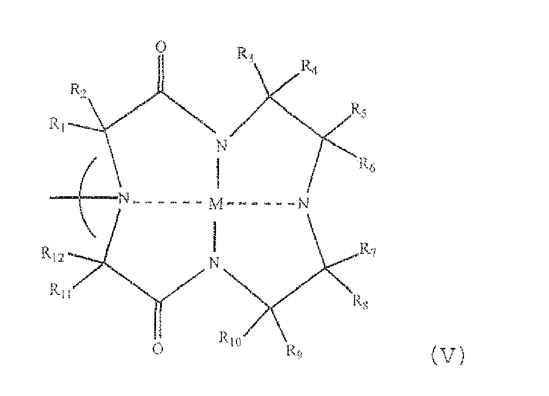
• or two polyazamacrocycles chosen from those corresponding to formulae (VI) and (VII) below: 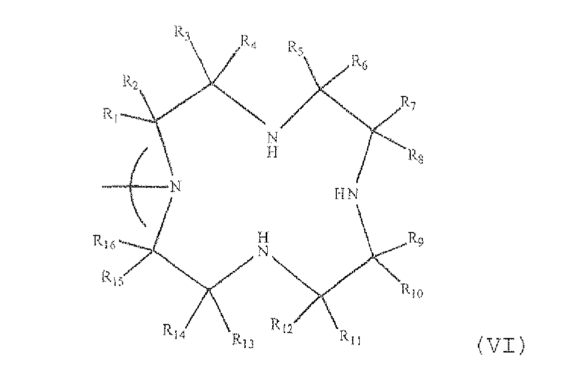 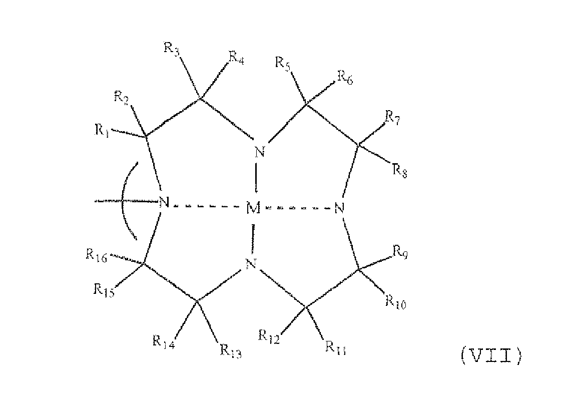 where R¹, R², R³, R⁴, R⁵, R⁶, R⁷, R⁸ R⁹, R¹⁰, R¹¹, R¹², R¹³, R¹⁴, R¹⁵, R¹⁶ and R¹⁷, being identical or different, represent a hydrogen atom or a linear or branched, saturated or unsaturated, C₁ to C₁₀ hydrocarbon group and M represents a metal chosen from lithium, magnesium, iron, cobalt, zinc, copper, nickel, manganese, chromium, titanium and lead;
p and q are identical, X and Y are identical; while E has the same meaning as before.

7. Use according to Claim 6, in which the compound corresponds to any one of the particular formulae (IIIa), (IIIb), (IIIc), (IVa), and (VIa) below:

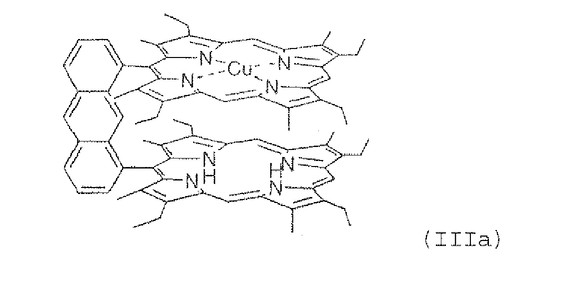

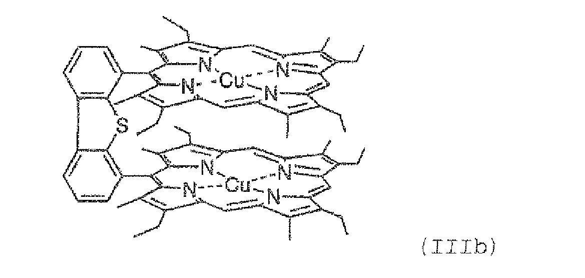

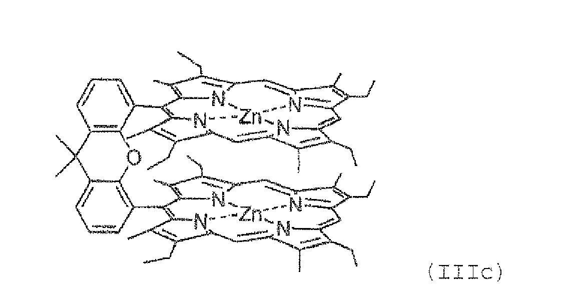

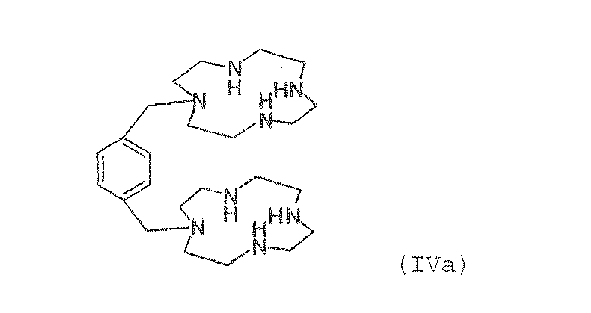

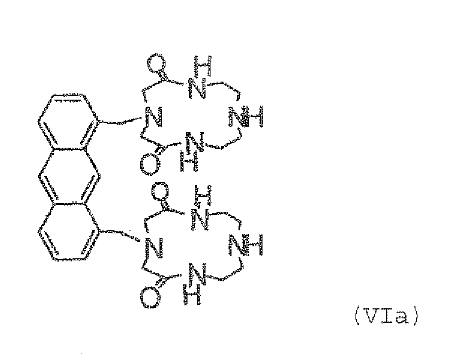

8. Use according to any one of the preceding claims, in which the compound is present in the sensor in the form of a thin film covering one or both sides of a substrate.

9. Use according to Claim 8, in which the thin film measures from 10 angstroms to 100 microns in thickness.

10. Use according to any one of the preceding claims, in which the sensor is a gravimetric sensor.

11. Use according to Claim 10, in which the gravimetric sensor is a quartz microbalance sensor.

12. Use according to any one of Claims 1 to 9, in which the sensor is a fluorescence sensor.

13. Use according to any one of Clams 1 to 9, in which the sensor is a multisensor which comprises one or more gravimetric sensors and/or one or more fluorescence sensors, at least one of these sensors comprising a compound corresponding to the general formula (I).

14. Use according to any one of the preceding claims, in which the organic compound or compounds to be detected, or assayed are one or more nitro compounds.

15. Use according to Claim 14, in which the nitro compound or compounds are chosen from nitroaromatic compounds, nitr amines, nitrosamines and citric esters.

16. Use according to Claim 15, in which the nitro compound or compounds are chosen from nitrobenzene, dinitrobenzene, trinitrobenzene, nitrotoluene, dinitrotoluene, trinitrotoluene, dinitrofluorobenzene, dinitrotrifluoromethoxybenzene, aminodinitrotoluene, dinitrotrifluoromethylbenzene, chlorodinitrotrifluoro-methylbenzene, hexanitrostilbene, trinitrophenol, cyclotetramethylenetetranitramine, cyclotrimethylenetrinitramine, trinitrophenylmethylnitramine, nitrosodimethylamine, pentrite, ethylene glycol dinitrate, diethylene glycol dinitrate, nitroglycerine or nitroguanidine.

17. Use according to any one of the preceding claims, in which the sensor is intended to detect explosives.

18. Use according to any one of Claims 1 to 13, in which the organic compound or compounds to be detected or assayed are one or more volatile organic compounds.

19. Use according to Claim 18, in which the volatile organic compound or compounds are chosen from ketones, alcohols, chlorinated solvents and aromatic compounds.

## Patentansprüche

1. Verwendung wenigstens einer der nachstehenden allgemeinen Formel (I) entsprechenden Verbindung 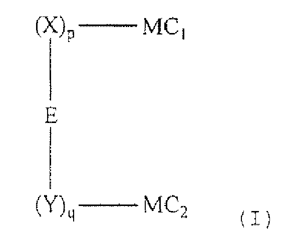 worin
MC₁ und MC₂, die gleich oder verschieden sein können, Makrocyclen darstellen, wobei sich diese Makrocyclen aus einem einzigen Cyclus oder mehreren Cyclen zusammensetzen, die durch eine Einfachbindung oder über ein Atom oder eine brückenbildende Gruppe miteinander verbunden sind, und deren Cyclus oder die Gesamtheit der Cyclen 8 bis 60 Kohlenstoffatome und ein oder mehrere Heteroatome umfassen;
p und q, die gleich oder verschieden sein können, 0 oder 1 bedeuten;
X und Y, die gleich oder verschieden sein können, 1 bis 10 Kohlenstoffatome umfassende Alkylengruppen sind, während
E eine cyclische oder heterocyclische Abstandsgruppe darstellt und
worin sich MC₁ und MC₂ einander gegenüberstehen, als empfindliches Material in einer chemischen Sonde zum Nachweisen oder Bestimmen einer oder mehrerer organischer Verbindungen im Dampfzustand.

2. Verwendung gemäß Anspruch 1, bei der die Makrocyclen MC₁ und MC₂ aus substituierten und unsubstituierten, metallierten und nicht metallierten Porphyrinen, Phthalocyaninen, Naphthalocyaninen, Sapphirinen, Corrolen, Corrolazinen und makrocyclischen Polyaminen ausgewählt sind.

3. Verwendung gemäß Anspruch 2, bei der die metallierten Porphyrine, Phthalocyanine, Naphthalocyanine, Sapphirine, Corrole, Corrolazine und makrocyclischen Polyamine Lithium, Magnesium, Eisen, Cobalt, Zink, Kupfer, Nickel, Mangan, Chrom, Titan oder Blei enthalten.

4. Verwendung gemäß einem der vorangehenden Ansprüche, bei der in der allgemeinen Formel (I) die Abstandsgruppe E aus Phenyl-, Pyrenyl-, Anthracenyl-, Naphthyl-, Dibenzofuranyl-, Biphenylyl-, Dibenzothiophenyl-, Xanthenyl-, Metallocenyl-, ortho-, meta- oder para-Xylyl-, Carbazolyl-, Acridinyl-, Phenazinyl-, Phenoxazinyl-, Binaphthyl-, Phenothiazinyl-, Fluorenyl-, Diphenyletheroxid- und Calix[n]arenylgruppen, worin n eine ganze Zahl von 4 bis 12 ist, ausgewählt ist.

5. Verwendung gemäß einem der vorangehenden Ansprüche, bei der die Verbindung der allgemeinen Formel (I) entspricht, worin
MC₁ und MC₂, die gleich oder verschieden sein können, zwei substituierte oder unsubstituierte, metallierte oder nicht metallierte Porphyrine oder zwei Phthalocyanine oder zwei Naphthalocyanine oder zwei Sapphirine oder zwei Corrole oder zwei Corrolazine oder zwei makrocyclische Polyamine darstellen,
p und q gleich sind und X und Y gleich sind, während E dieselbe Bedeutung wie voranstehend aufweist.

6. Verwendung gemäß Anspruch 5, bei der die Verbindung der allgemeinen Formel (I) entspricht, worin
MC₁ und MC₂ Folgendes darstellen:
• entweder zwei Porphyrine, die aus denen ausgewählt sind, die den nachstehenden Formeln (II) und (III) entsprechen: 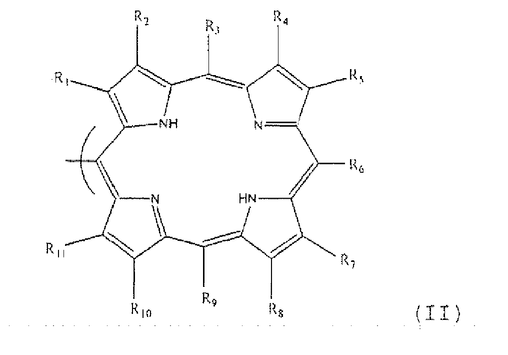 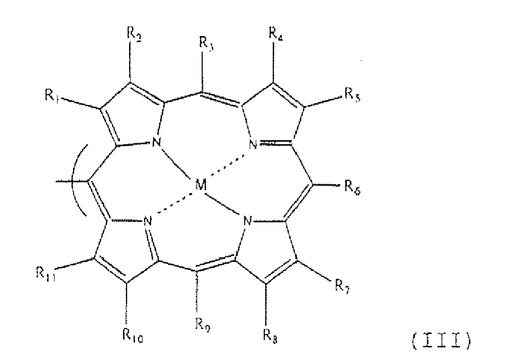
• oder zwei Dioxopolyazamakrocyclen, die aus denen ausgewählt sind, die den nachstehenden Formeln (IV) und (V) entsprechen: 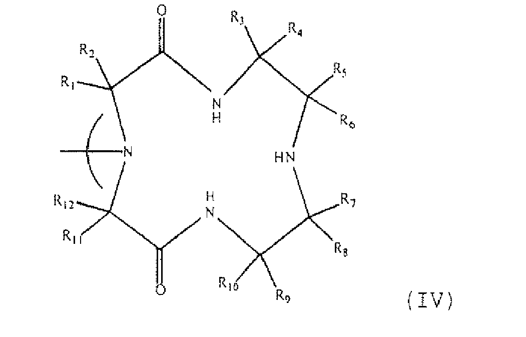 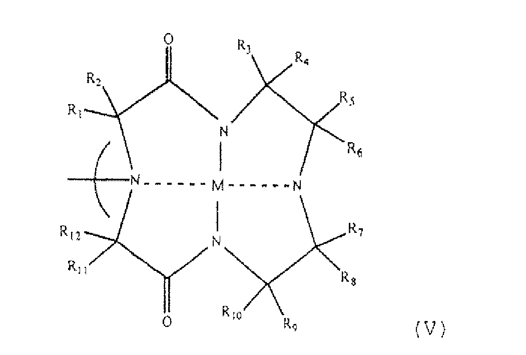
• oder zwei Polyazamakrocyclen, die aus denen ausgewählt sind, die den nachstehenden Formeln (VI) und (VII) entsprechen: 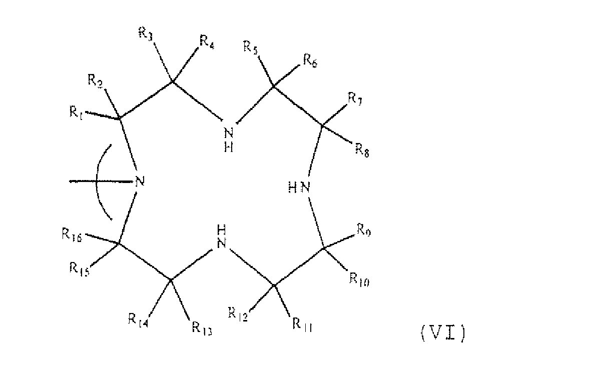 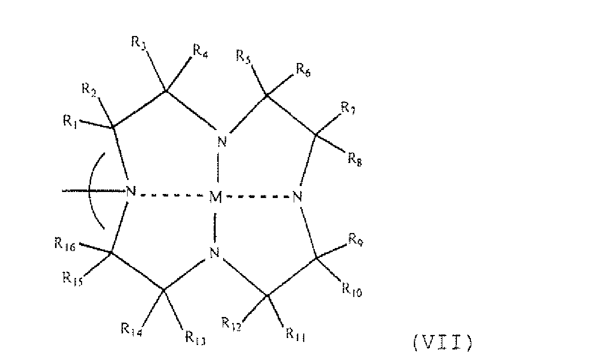
worin R¹, R², R³, R⁴, R⁵, R⁶, R⁷, R⁸, R⁹, R¹⁰, R¹¹, R¹², R¹³, R¹⁴, R¹⁵, R¹⁶ und R¹⁷, die gleich oder verschieden sind, ein Wasserstoffatom oder eine gesättigte oder ungesättigte, gerade oder verzweigte C₁-C₁₀-Kohlenwasserstoffgruppe bedeuten und M ein aus Lithium, Magnesium, Eisen, Cobalt, Zink, Kupfer, Nickel, Mangan, Chrom, Titan und Blei ausgewähltes Metall darstellt,
p und q identisch sind und X und Y identisch sind, während E dieselbe Bedeutung wie voranstehend aufweist.

7. Verwendung gemäß Anspruch 6, bei der die Verbindung einer der nachfolgenden speziellen Formeln (IIIa), (IIIb), (IIIc), (IVa) und (VIa) entspricht:

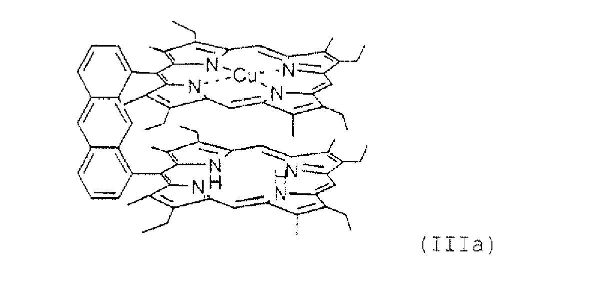

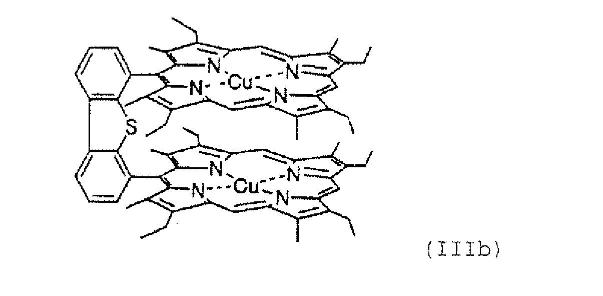

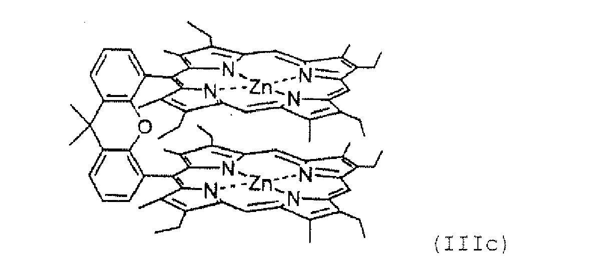

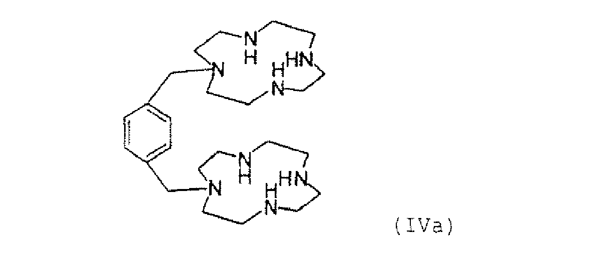

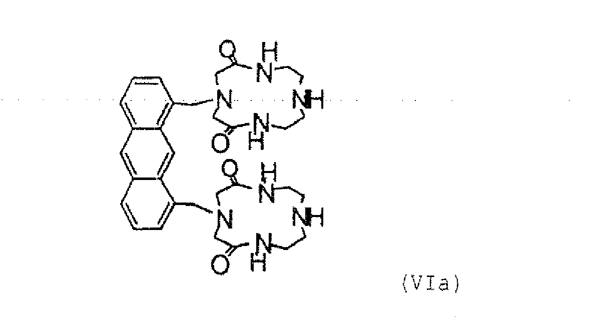

8. Verwendung gemäß einem der vorangehenden Ansprüche, bei der die Verbindung in der Sonde in Form eines eine oder beide Seiten eines Substrats bedeckenden Films vorliegt.

9. Verwendung gemäß Anspruch 8, bei der der dünne Film in der Dicke 10 Ångström bis 100 Mikron misst.

10. Verwendung gemäß einem der vorangehenden Ansprüche, bei der die Sonde eine gravimetrische Sonde ist.

11. Verwendung gemäß Anspruch 10, bei der die gravimetrische Sonde eine Quarzmikrowaagensonde ist.

12. Verwendung gemäß einem der Ansprüche 1 bis 9, bei der die Sonde eine Fluoreszenzsonde ist.

13. Verwendung gemäß einem der Ansprüche 1 bis 9, bei der die Sonde eine Multisonde ist, die eine oder mehrere gravimetrische Sonden und/oder eine oder mehrere Fluoreszenzsonden umfasst, wobei wenigstens eine dieser Sonden eine der allgemeinen Formel (I) entsprechende Verbindung umfasst.

14. Verwendung gemäß einem der vorangehenden Ansprüche, bei der die nachzuweisende oder zu bestimmende organische Verbindung oder Verbindungen eine Nitroverbindung oder Nitroverbindungen sind.

15. Verwendung gemäß Anspruch 14, bei der die Nitroverbindung oder Nitroverbindungen aus aromatischen Nitroverbindungen, Nitraminen, Nitrosaminen und Salpetersäureestern ausgewählt sind.

16. Verwendung gemäß Anspruch 15, bei der die Nitroverbindung oder Nitroverbindungen aus Nitrobenzol, Dinitrobenzol, Trinitrobenzol, Nitrotoluol, Dinitrotoluol, Trinitrotoluol, Dinitrofluorbenzol, Dinitrotrifluormethoxybenzol, Aminodinitrotoluol, Dinitrotrifluormethylbenzol, Chlordinitrotrifluormethylbenzol, Hexanitrostilben, Trinitrophenol, Cyclotetramethylentetranitramin, Cyclotrimethylentrinitramin, Trinitrophenylmethylnitramin, Nitrosodimethylamin, Pentrit, Ethylenglykoldinitrat, Diethylenglykoldinitrat, Nitroglycerin oder Nitroguanidin ausgewählt sind.

17. Verwendung gemäß einem der vorangehenden Ansprüche, bei der die Sonde zum Nachweisen von Sprengstoffen bestimmt ist.

18. Verwendung gemäß einem der Ansprüche 1 bis 13, bei der die nachzuweisende oder zu bestimmende Verbindung oder Verbindungen eine flüchtige organische Verbindung oder Verbindungen sind.

19. Verwendung gemäß Anspruch 18, bei der die flüchtige organische Verbindung oder Verbindungen aus Ketonen, Alkoholen, chlorierten Lösungsmitteln und aromatischen Verbindungen ausgewählt sind.
